# EUROPÄISCHE PATENTSCHRIFT

(11) **EP 2 688 517 B1**
(45) Veröffentlichungstag und Bekanntmachung des Hinweises auf die Patenterteilung: **25.07.2018**
(21) Anmeldenummer: 12709644.4
(22) Anmeldetag: 21.03.2012
(51) Int. Cl.: A61F 2/34, A61F 2/30

(54) **GELENKPFANNENIMPLANTAT**
BALL-AND-SOCKET IMPLANT
IMPLANT À COQUE ACÉTABULAIRE

(30) Priorität: 21.03.2011 EP 11159050
(43) Veröffentlichungstag der Anmeldung: 29.01.2014
(73) Patentinhaber: Jossi Holding AG, 8546 Islikon (CH)
(72) Erfinder: GUGLER, Christian, CH-8500 Frauenfeld (CH); SCHMIDT, Martin, CH-9545 Wängi (CH); MEYENHOFER, Andreas, CH-8255 Schlattingen (CH)
(74) Vertreter: Hepp Wenger Ryffel AG
(86) Internationale Anmeldenummer: PCT/EP2012/054991
(87) Internationale Veröffentlichungsnummer: WO 2012/126944

(56) Entgegenhaltungen:
- EP-A1- 2 338 443
- EP-B1- 0 839 016
- WO-A1-97/39702
- DE-A1- 2 259 313
- DE-A1- 10 106 863
- DE-C1- 3 535 959
- GB-A- 2 268 408

## Beschreibung

Die vorliegende Erfindung betrifft ein Gelenkpfannenimplantat gemäss dem Oberbegriff von Anspruch 1.

Zur besseren Verankerung von Implantaten, insbesondere Endoprothesen in Knochen sind eine Vielzahl von Oberflächenstrukturen bekannt. Diese reichen von aufgerauten Oberflächen bis zur Ausstattung der Oberfläche mit komplexen Strukturelementen.

Insbesondere im Bereich der Hüftgelenksprothesen ist es von Vorteil, wenn die Implantatteile, vor allem die im Hüftknochen angebrachte Gelenkpfanne, ausreiss- und drehfest im Knochen verankert werden können. Während eine Gruppe von Gelenkpfannen durch Schrauben verankert wird, erfolgt bei einer zweite Gruppe die Befestigung mittels Knochenzement oder durch Einschlagen.

Um das Ausreissverhalten sowie die Drehfestigkeit einer mittels Knochenzement oder Einschlagen befestigten Gelenkpfanne zu erhöhen, wird die Oberfläche solcher Pfannen üblicherweise mit Strukturelementen versehen. Diese Strukturelemente verteilen dabei auftretende Kräfte gleichmässig und verhindern so eine Drehung oder gar das Ausreissen der Gelenkpfanne. Im Falle des Einschlagens gewährleisten die Strukturelemente ein gutes Einwachsen in den Knochen.

Dem Fachmann ist eine Vielzahl an verschiedenen Oberflächenstrukturen bekannt. Die Oberflächenstrukturen werden dabei meistens durch spanabhebende Verfahren in die Oberfläche der Implantate eingearbeitet.

Die EP 0 839 016 offenbart beispielsweise eine Hüftgelenkpfanne mit einer Oberflächenstruktur mit rhombenförmigen Erhebungen.

Die Erhebungen werden durch sich überkreuzende, schraubenlinienförmige Gewinde mit entgegengesetzter Steigung begrenzt. Die Tiefe der Gewinde nimmt dabei vom Äquator zum Pol hin ab.

Die DE 101 06 863 beschreibt eine implantierbare Hüftpfanne welche an der Oberfläche eine Struktur in Form von Pyramidenspitzen aufweist. Die Pyramidenspitzen werden durch spanabhebendes Einarbeiten von Rechtsgewinde-Profilen und Linkgsgewinde-Profilen erzeugt. Die Profilhöhe dieser Gewindeprofile nimmt von der Eingangsebene der Pfanne gegen deren Polbereich hin ab.

Die GB 2 268 408 beschreibt ein orthopädisches Implantat zum Einpressen mit einer hemisphärischen Aussenfläche, auf welcher ein reguläres Muster aufgeprägt ist. Das Muster umfasst pyramidenförmige Strukturelemente, welche von mit entgegengesetzter Steigung verlaufenden Rillen gebildet sind.

Die EP 2 338 443 beschreibt eine künstliche Hüftgelenkpfanne zur unzementierten Verankerung. Auf der Aussenseite trägt die Halbschale eine Zahnstruktur, die ringförmig angeordnet ist. Die äquatorwärts orientierte Flanke der einzelnen Zähne ergibt einen Widerhaken-Effekt.

Die WO97/39702 beschreibt eine einschraubbare Hüftgelenkpfanne mit einem speziellen Gewinde. Durch die spezielle Gewindekonfiguration soll ein kraftsparendes Einschrauben gewährleistet werden.

Die DE 35 35 959 betrifft eine Hüftgelenkpfanne an deren Aussenschale ein selbstschneidendes Gewinde zur zementfreien Verankerung angeordnet ist. Der Aussendurchmesser des Gewindes verläuft annähernd über alle Gewindegänge hinweg konstant.

Die DE 2 259 313 betrifft eine Knochenprothese aus gesinterter Tonerde. Auf der Aussenseite der Prothese sind Unebenheiten angeordnet, die durch Rillen gebildet sind. Diese verlaufen längs der Breitenkreise und der Meridiane der Halbschale.

Durch die Kombination von Gewinde und Kugelkalotte nimmt die Anzahl der Erhebungen gegen den Pol hin stark ab, was die Verankerung der Gelenkspfanne im Knochen reduziert. Ferner wird durch die abnehmende Tiefe der Gewinde die Verankerung im Knochen zusätzlich geschwächt.

Eine Aufgabe der vorliegenden Erfindung ist es, eine Oberflächenstruktur für Gelenkpfannenimplantate zu schaffen, welche die Nachteile des Bekannten überwindet und welche insbesondere eine gute Verankerung der Gelenkpfanne im Knochen ermöglicht und einfach herzustellen ist. Diese Aufgabe wird mit einem Gelenkpfannenimplantat gemäss Anspruch 1 gelöst.

Das erfindungsgemässe Gelenkpfannenimplantat hat vorzugsweise die Form einer Kugelkalottenschale und weist mindestens einen Bereich mit einer Oberflächenstruktur auf. Der mindestens eine Bereich erstreckt sich zwischen dem Äquator und dem Pol der Kugelkalottenschale. Die Oberflächenstruktur umfasst eine Vielzahl von Strukturelementen, welche durch jeweils mehrere, sich überkreuzende Rillen mit entgegengesetzter Steigung gebildet sind. Die Anzahl der Strukturelemente auf dem Äquator oder einem äquatornahen Breitenkreis ist dabei gleich wie auf einem polnahen Breitenkreis. Die Rillen weisen eine Krümmung derart auf, dass die Steigung der Rillen gegenüber den Breitenkreisen und somit ein Winkel zwischen einer Tangente der Rillenkrümmung und einem Breitenkreis vom Äquator zum Pol hin kontinuierlich zunimmt.

Insbesondere heisst dies, dass die Rillen einen Verlauf derart aufweisen, dass die Steigung der Rillen gegenüber den Breitenkreisen und somit ein Winkel zwischen einer Tangente des Rillenverlaufs und einem Breitenkreis vom Äquator zum Pol hin kontinuierlich zunimmt. Es versteht sich, dass der Winkel zwischen Tangente des Rillenverlaufs und einem Breitenkreis vom Äquator zum Pol hin dabei nicht zwingend stetig zuzunehmen braucht.

Mit Krümmung ist hierbei ein Verlauf der Richtungsänderungen beim Durchlaufen der Kurve bezeichnet. Die vorliegend anspruchsgemässe Krümmung versteht sich hierbei als derjenige zusätzliche Krümmungsanteil, welcher über eine Krümmung hinausgeht, die durch die gekrümmte Oberfläche, auf welcher die Rillen ausgebildet sind, vorgegeben ist. Der Verlauf der Rillen des erfindungsgemässen Gelenkpfannenimplantats ist von Kugelloxodromen zu unterscheiden, welche die Breitenkreise immer unter dem gleichen Winkel schneiden, also eine konstante Steigung aufweisen (so aber dennoch aufgrund der gekrümmten Oberfläche eine vorgegebene räumliche Krümmung haben).

Das erfindungsgemässe Gelenkpfannenimplantat ist vorzugsweise ein Hüftgelenkpfannenimplantat. Das Gelenkpfannenimplantat weist bevorzugterweise im Wesentlichen die Form einer Kugelkalottenschale auf, besonders bevorzugt die Form einer Halbkugelschale. Es versteht sich, dass die Kugelkalottenschale Abweichungen von einer reinen Kugelform aufweisen kann. Insbesondere kann die Kalotte z.B. im Polbereich eine Vertiefung bzw. eine Bohrung zu Manipulationszwecken aufweisen. Ebenso kann die Kalotte im Polbereich abgeflacht sein, um beim Implantieren ein erwünschtes Verklemmen der Schale massgeblich im äquatorialen Bereich zu begünstigen.

Die Kugelkalottenschale weist eine äussere, konvexe Oberfläche auf, welche bei der im Knochen eingesetzten Gelenkpfanne auf dem Knochen anliegt. Weiter weist die Kugelkalottenschale eine innere, konkave oder annähernd konkave Oberfläche auf, in die eine Lagerschale eingesetzt werden kann. Zwischen diesen beiden Oberflächen weist das Gelenkpfannenimplantat eine Wand auf, deren Dicke vorzugsweise überall gleich ist, d.h. die Radien der äusseren wie auch der inneren Oberfläche haben den gleichen Mittelpunkt und sind beide im Wesentlichen konstant. Alternativ kann sich die Dicke der Wand auch verändern, z.B. kann die innere Oberfläche aus mehreren in spezifischen Winkel zueinander stehenden Flächen bestehen oder die äussere Oberfläche eine Äbflachung im Polbereich aufweisen oder die äussere Oberfläche eine Abflachung im Polbereich aufweisen.

Die Grösse der Kugelkalottenschale kann je nach Patient variieren, für den das Gelenkpfannenimplantat vorgesehen ist oder je nach Anwendungsort, beispielsweise am Hüft- oder Schultergelenk.

Ferner weist das Gelenkpfannenimplantat mindestens einen Bereich mit einer Oberflächenstruktur auf. Dieser Bereich befindet sich zwischen dem Äquator und dem Pol und erstreckt sich bevorzugterweise um den gesamten Umfang des Gelenkpfannenimplantats. Je nach Ausgestaltung weist der Bereich unterschiedliche Ausdehnungen zwischen Äquator und Pol des Gelenkpfannenimplantats auf. Besonders bevorzug erstreckt sich dieser Bereich vom Äquator in Richtung Pol über mindestens die Hälfte der Oberfläche.

Als "Äquator" im Sinne der Anmeldung wird die Öffnung der Kugelkalottenschale verstanden, während als "Pol" diejenige Stelle bezeichnet wird, welche dem Apex der Kugelkalottenschale entspricht.

Ein "äquatornaher Bereich" im Sinne der vorliegenden Anmeldung liegt näher am Äquator als am Pol, während ein "polnaher Bereich" näher beim Pol als beim Äquator des Gelenkpfannenimplantats liegt.

Die Oberflächenstruktur umfasst eine Vielzahl von Strukturelementen. Die Strukturelemente werden durch sich überkreuzende Rillen gebildet. Die Rillen sind bevorzugt durch ein spanabhebendes Verfahren in der Oberfläche erzeugt worden, wie beispielsweise durch Fräsen oder Stossen.

Die Rillen weisen bevorzugt entgegengesetzte Steigungen auf, d.h. weisen bezüglich einer Polachse der Kugelkalottenschale eine entgegengesetzte Umlaufrichtung auf. Mit anderen Worten verlaufen in Bezug auf den Äquator einzelne Rillen nach links während andere nach rechts verlaufen. Jedes Strukturelement ist von zwei benachbarten nach links und zwei benachbarten nach rechts verlaufenden, sich kreuzenden Rillen begrenzt. Jedes Strukturelement weist damit einen im Wesentlichen rhombenförmigen Grundfläche bzw. Grundriss auf, wenn von der bezogen auf die Grösse der Strukturelemente vernachlässigbaren Krümmung der Begrenzungen aufgrund der Rillenkrümmung abgesehen wird.

Die Rillen sind bevorzugt derart ausgebildet, dass die Anzahl der Strukturelemente auf dem Äquator oder einem äquatornahen Breitenkreis gleich ist wie auf einem polnahen Breitenkreis. Die Anzahl der Strukturelemente auf jedem beliebigen Breitenkreis des Gelenkpfannenimplantats innerhalb eines Bereichs mit Oberflächenstruktur ist daher gleich.

Die Rillen mit der gleichen Steigungsrichtung, d.h. mit der gleichen Umlaufrichtung, weisen bevorzugt alle dieselbe Krümmung bzw. denselben Krümmungsverlauf auf. Es versteht sich, dass, je nach Erfordernis, die Rillen mit unterschiedlicher Umlaufrichtung, d.h. mit entgegengesetzter Steigungsrichtung, mit Vorteil auch eine unterschiedliche Krümmung bzw. einen unterschiedlichen Verlauf aufweisen können. Z.B. können bei einem bestimmten Breitenkreis die Rillen mit gleicher Steigungsrichtung eine Steigung haben, welche sich von einer Steigung der Rillen mit entgegengesetzter Steigungsrichtung (auch betragsmässig) unterscheidet.

Damit die Anzahl der Strukturelemente auf dem Äquator oder einem äquatornahen Breitenkreis gleich ist wie auf einem polnahen Breitenkreis, nimmt die Steigung der Rillen gegenüber den Breitenkreisen und somit der Winkel zwischen einer Tangente der Rillenkrümmung bzw. des Rillenverlaufs und einem Breitenkreis vom Äquator zum Pol hin kontinuierlich, aber nicht zwingend stetig, zu.

Gleichzeitig wird der Radius der Krümmung vom Äquator zum Pol hin bevorzugt kontinuierlich kleiner, d.h. die Krümmung der Rille nimmt zum Pol hin kontinuierlich zu. Der Steigungsverlauf kann derart gewählt sein, dass der Abstand zwischen zwei Rillen mit gleicher Steigungsrichtung vom Äquator in Richtung zum Pol hin konstant bleibt.

Der Grundriss der Strukturelemente verändert sich vom Pol zum Äquator hin. Die Strukturelemente, welche auf dem gleichen Meridian liegen, weisen aufgrund der zum Pol hin zunehmenden Steigung der Rillen eine vom Pol in Richtung Äquator stetig grösser werdende Ausdehnung parallel zu den Breitenkreisen auf. Eine Ausdehnung in Richtung senkrecht zu den Breitenkreisen verringert sich hingegen.

Der Steigungsverlauf der Rillen kann so gewählt werden, dass ein Flächeninhalt der Grundrissfläche der Strukturelemente in Äquator- und Polnähe möglichst ähnlich bzw. in etwa gleich ist, damit sich gesamthaft eine optisch weitgehend homogene Oberflächestruktur ergibt.

Da die Gelenkpfanne in einer Richtung rechtwinklig zu den Breitenkreisen in den Knochen eingesetzt wird, hat dies den Vorteil, dass sich jedes Strukturelement in Richtung parallel zu den Breitenkreisen ein bisschen weiter in den Knochen schneiden muss, was die Verankerung des Gelenkpfannenimplantats im Knochen erhöht.

Bevorzugt weisen zwei benachbarte Rillen mit gleicher Steigungsrichtung bzw. Umlaufrichtung über ihre gesamte Länge einen weitgehend konstanten Abstand zueinander auf. Dies kann durch eine geeignete Wahl des Steigungsverlaufs der Rillen als Funktion des Abstands des jeweiligen Breitenkreises vom Äquator erreicht werden. Bevorzugt weisen benachbarte Rillen mit gleicher Steigungsrichtung paarweise, d.h. jedes beliebige benachbarte Paar, denselben Abstand auf.

Es versteht sich, dass sich die Rillen aufgrund des konstanten Abstandes nicht gänzlich bis zum Pol erstrecken können, sondern nur bis zu einem polnächsten Breitenkreis, nämlich dort, wo der Steigungswinkel zwischen Rillen und Breitenkreis 90° erreicht. Der polnächste mögliche Breitenkreis bestimmt sich dabei aufgrund einer Reihe von Faktoren wie beispielsweise Abstand der Rillen, Anzahl der Rillen, Breite der Rillen etc.

Bevorzugt weisen die Rillen vom äquatornahen Bereich zum polnahen Bereich hin im mindestens einen Bereich eine konstante Tiefe auf. Dadurch weisen auch die Strukturelemente eine konstante Höhe auf. Dadurch ist die Verankerung über der gesamten Oberfläche des Gelenkpfannenimplantats gleich gut. Ausserdem ermöglicht eine solche Konfiguration eine gleich gute Stabilität des Gelenkpfannenimplantats im Knochen entlang der Polachse wie auch gegen Rotation und Verkippen.

Bevorzugt ist der Winkel zwischen einer Tangente der Rillenkrümmung bzw. des Rillenverlaufs jeder Rille und einem bestimmten Breitenkreis betragsmässig gleich. Damit ergibt sich eine besonders regelmässige Ausbildung der Strukturelemente und damit auch der Oberflächenstruktur, was eine besonders gleichmässige Verankerung des Gelenkpfannenimplantats z.B. durch Einwachsen in den Knochen oder Verankerung im Knochenzement ermöglicht.

Mit Vorteil sind die Strukturelemente durch benachbarte Rillen unterschiedlicher Flankenform, Rillentiefe, Rillenbreite, Rillenabstand und der Rillenzahl konfiguriert.

Die Strukturelemente sind besonders bevorzugt in der Form einer Pyramide oder eines Pyramidenstumpfes ausgebildet. Die Form der Strukturelemente ist durch das Profil der Rillen vorgegeben, so dass sich durch geeignete Wahl des Rillenprofils die Form der Strukturelemente verändern lässt. Insbesondere kann eine Flankenform der Strukturelemente durch das Profil der Rillen vorgegeben werden. Alternativ weisen die Strukturelemente eine beliebige polyedrische oder auch gerundete Form auf. Nach dem Erzeugen der Strukturelemente durch die Rillen können diese noch nachbearbeitet werden, z.B. durch Fräsen oder durch umformende Verfahren.

Bevorzugt sind die Strukturelemente im meridianen Querschnitt in Richtung Äquator geneigt. D.h. dass mindestens diejenige Seite des Strukturelements, welche in Richtung Äquator zeigt, steiler ansteigt oder gar überhängt. Der damit erreichte sägezahnartige Querschnitt der Strukturelemente längs eines Meridians erhöht die zum Ausreissen des Gelenkpfannenimplantats nötige Kraft zusätzlich.

Bevorzugt weist das Gelenkpfannenimplantat mindestens einen zweiten Bereich mit einer Oberflächenstruktur auf. Bevorzugt umfassen die Oberflächenstrukturen in allen Bereichen eine Vielzahl von Strukturelementen, welche weitgehend analog dem ersten Bereich durch jeweils mehrere, sich überkreuzende Rillen mit entgegen gesetzter Steigung gebildet sind. Die Oberflächenstrukturen der Bereiche weisen dabei vorzugsweise eine unterschiedliche Konfiguration auf (z.B. Tiefe der Rillen, Anzahl der Rillen, Verlauf der Rillen, Profil der Rillen, Abstand der Rillen etc.). Die beiden Bereiche mit Oberflächenstrukturen weisen mit Vorteil eine unterschiedliche Anzahl von Strukturelementen und/oder Rillen auf und/oder die Strukturelemente unterscheiden sich in ihrer Konfiguration (z.B. Flankenform oder Tiefe der Rillen). Ferner bevorzugt kann das Gelenkpfannenimplantat auch mehr als zwei Bereiche mit unterschiedlichen Oberflächenstrukturen aufweisen, wie zum Beispiel drei, vier, fünf oder mehr Bereiche. Die Bereiche können alle unterschiedliche Ausdehnungen zwischen Äquator und Pol des Gelenkpfannenimplantats aufweisen oder aber alle die gleiche Ausdehnung. Dadurch kann das Gelenkpfannenimplantat mit Bereichen ausgestattet werden, welche an spezifische Knochenbereiche angepasste Verankerungseigenschaften aufweisen.

Besonders bevorzugt umfasst das erfindungsgemässe Gelenkpfannenimplantat den ersten Bereich mit einer ersten Oberflächenstruktur mit Strukturelementen mit im meridianen Querschnitt in Richtung Äquator geneigten Oberflächenstrukturen in einem äquatornahen Bereich und den zweiten Bereich mit einer zweiten Oberflächenstruktur mit Strukturelementen in Form von Pyramiden in einem polnahen Bereich.

Eine derartige Konfiguration des Gelenkpfannenimplantats hat den Vorteil, dass der äquatornahe Bereich durch die sägezahnförmigen Strukturelemente die zum Ausreissen des eingesetzten Gelenkpfannenimplantats nötige Kraft zusätzlich erhöht, während die pyramidenförmigen Strukturelemente im polnahen Bereich das Gelenkpfannenimplantat optimal vor dem Verdrehen und/oder Verkippen sichern und ein optimales Einwachsen in den Knochen auch in diesem Bereich sicherstellen.

Bevorzugterweise bilden die Rillen in einem äquatornahen Bereich der Oberflächenstruktur mit einem Breitenkreis einen Winkel von kleiner als 90°. Bevorzugt liegt dieser Winkel in einem Bereich von 10° bis 60°, besonders bevorzugt in einem Bereich von 15° bis 50°. Durch die Krümmung der Rillen, d.h. durch den Steigungsverlauf, vergrössert sich dieser Winkel zum Pol hin kontinuierlich.

Der mindestens eine Bereich mit einer Oberflächenstruktur weist bevorzugt auf dem Äquator oder dem äquatornahen Breitenkreis sowie dem polnahen Breitenkreis 10 bis 100 Strukturelemente auf.

Bevorzugt weisen die Strukturelemente Grundrisse mit Seitenlängen von etwa 0.2 - 5 mm auf.

Besonders bevorzugt weisen die Strukturelemente einen Grundriss auf, welcher annähernd rhombenförmig ist. Im Unterschied zu einem Rhombus sind die Seitenkanten, d.h. die die Grundfläche begrenzenden Rillen nicht gerade sondern weisen eine Krümmung auf. Näherungsweise kann zur Vereinfachung jedoch von einer Rhombenform ausgegangen werden. Durch die Krümmung bzw. den Verlauf der Rillen nimmt die Länge der zu den Meridianen parallelen Diagonalen der Grundflächen der Strukturelemente vom Äquator zum Pol hin zu während die zu den Breitenkreisen parallelen Diagonalen abnehmen. D.h. dass ein Strukturelement je nach Nähe zum Äquator beziehungsweise zum Pol einen unterschiedlichen Grundriss aufweist.

Die Strukturelemente, welche näher beim Pol sind, weisen einen Grundriss auf, welcher in Richtung der Meridiane länger ist als in Richtung der Breitenkreise. Die Form der Grundflächen d.h. der Grundriss erleichtert dabei das Einsetzen des Gelenkpfannenimplantats, da sich die Strukturelemente leichter in den Knochen einschneiden können.

Die unterschiedlichen Geometrien der Grundrisse der Strukturelemente weisen zudem den Vorteil auf, dass diejenigen Strukturelemente, welche eine grössere Ausdehnung in Richtung der Meridiane aufweisen das Gelenkpfannenimplantat gut gegen Verdrehen und Verkippen sichern, während diejenigen Strukturelemente, welche in Richtung der Breitenkreise eine grössere Ausdehnung aufweisen, das Gelenkpfannenimplantat besser gegen das Ausreissen sichern.

Bevorzugterweise weisen die Rillen einen symmetrischen oder asymmetrischen Querschnitt auf. Dadurch lässt sich die Form der Strukturelemente, insbesondere die Flankenform, zusätzlich verändern.

Weitere Aspekte sowie Details der Erfindung ergeben sich aus der folgenden Beschreibung von Beispielen und Figuren. Es zeigen:
- Fig. 1:: eine Aufsicht eines Zwischenerzeugnisses bei der Produktion eines Gelenkpfannenimplantats mit Rillen;
- Fig. 2:: eine Seitenansicht des Zwischenerzeugnisses der Fig. 1;
- Fig. 3:: eine Aufsicht eines erfindungsgemässen Gelenkpfannenimplantats mit einem Bereich mit einer Oberflächenstruktur;
- Fig. 4:: eine Seitenansicht des Gelenkpfannenimplantats der Figur 3;
- Fig. 5:: eine Aufsicht eines Gelenkpfannenimplantats mit zwei Bereichen mit jeweils unterschiedlichen Oberflächenstrukturen;
- Fig. 6:: eine Seitenansicht des Gelenkpfannenimplantats der Fig. 4;
- Fig. 7:: einen Teilschnitt durch das Gelenkpfannenimplantat der Figur 4; und
- Fig. 8:: eine qualitative Mercator-Projektion zweier Rillen mit entgegen gesetzter Steigungsrichtung auf der Oberfläche eines erfindungsgemässen Gelenkpfannenimplantats;
- Fig. 9:: eine Schrägansicht eines Zwischenerzeugnisses bei der Herstellung eines Gelenkpfannenimplantats mit einzelnen Rillen;
- Fig. 10:: eine Seitenansicht einer Kalottenschale für eine weitere Ausführung eines erfindungsgemässen Gelenkpfannenimplantats mit eingezeichneten Rillenverlauf;
- Fig. 11:: eine Aufsicht auf die Kalottenschale gemäss Fig. 10;
- Fig. 12:: eine fotografische Seitenansicht eines erfindungsgemässen Gelenkpfannenimplantats.

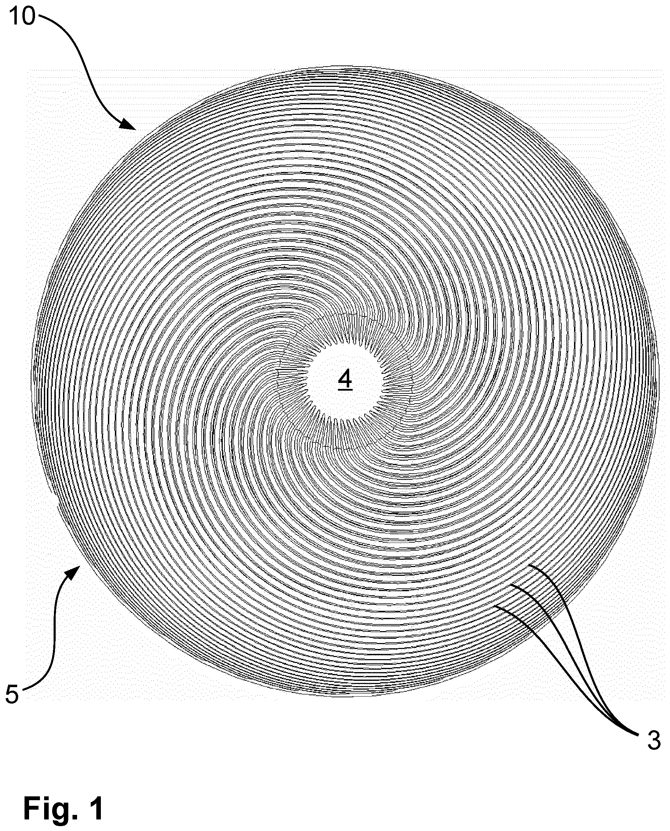

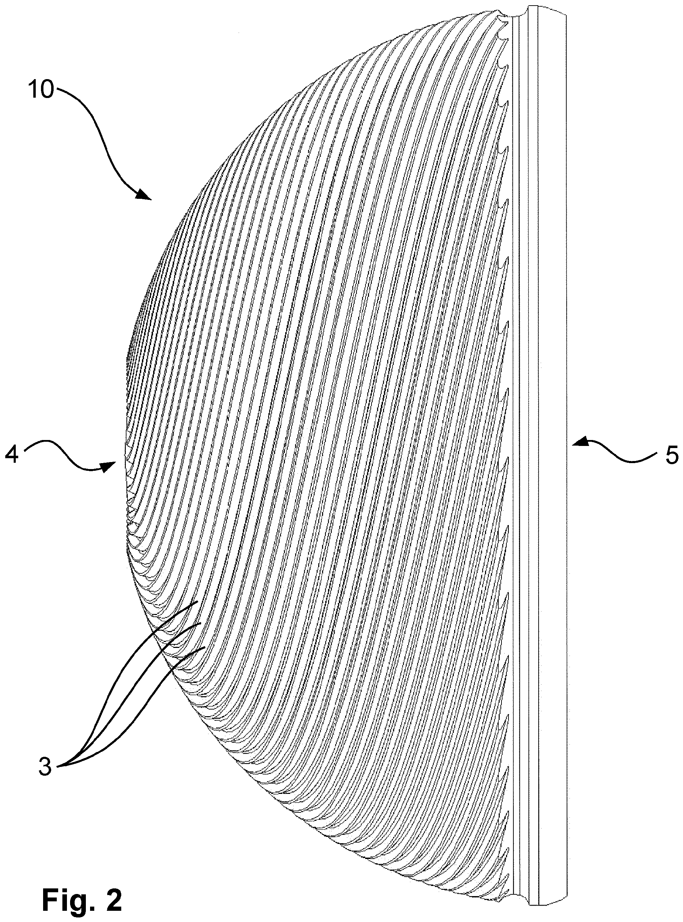

Die Figur 1 zeigt eine Aufsicht auf ein Zwischenerzeugnis 10 bei der Herstellung eines Gelenkpfannenimplantats 1 mit nur nach Links gerichteten Rillen 3, d.h. linkshändiger Umlaufrichtung. Die Figur 2 zeigt eine Seitenansicht desselben Zwischenerzeugnisses 10. Das Zwischenerzeugnis 10 ist in der Form einer Kugelkalottenschale und weist einen Pol 4 sowie einen Äquator 5 auf. Bei diesem Ausführungsbeispiel entspricht die Kugelkalottenschale einer Halbkugelschale. Das Zwischenerzeugnis 10 ist das Ergebnis eines ersten Bearbeitungsschrittes bei der Herstellung einer erfindungsgemässen Gelenkpfannenimplantats, bei dem zunächst sämtliche Rillen 3 in nur einer Steigungsrichtung eingearbeitet werden.

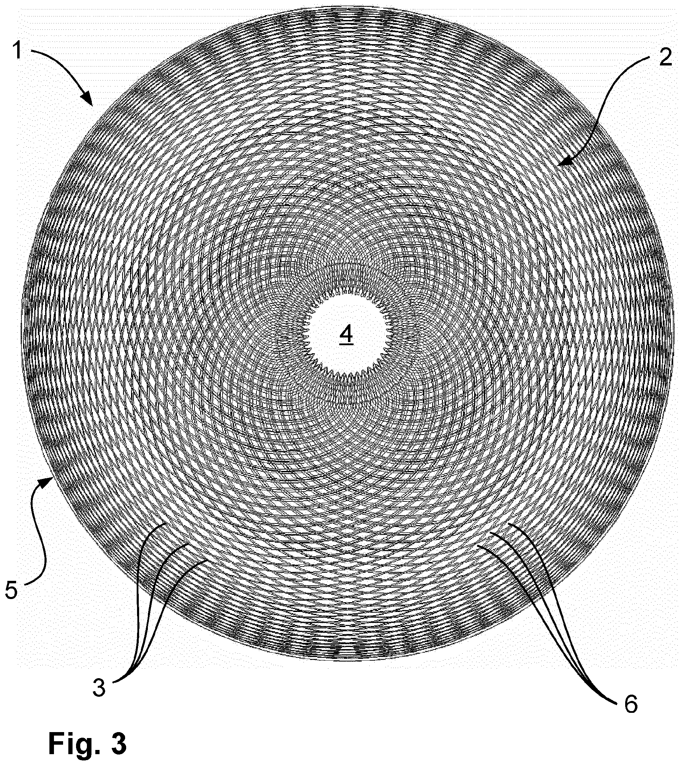

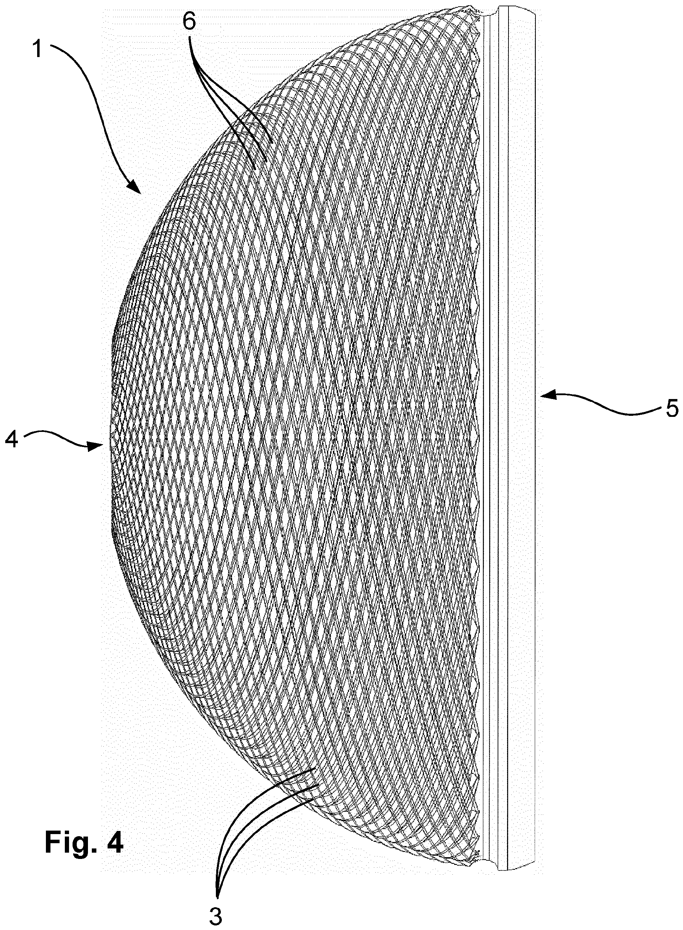

Die Figuren 3 und 4 zeigen eine Auf- sowie eine Seitenaufsicht eines Gelenkpfannenimplantats 1 mit einem Bereich mit einer Oberflächenstruktur 2. Die Oberflächenstruktur 2 weist eine Vielzahl von Strukturelementen 6 auf, welche durch die sich überkreuzenden Rillen 3 gebildet sind. Die Rillen 3 weisen eine Krümmung bzw. einen Verlauf derart auf, dass die Steigung der Rillen 3 gegenüber den Breitenkreisen und somit ein Winkel zwischen einer Tangente der Rillenkrümmung und einem Breitenkreis vom Äquator 5 zum Pol 4 hin kontinuierlich zunimmt. Die Rillen 3 sind derart gewählt, dass die Anzahl der Strukturelemente 6 auf dem Äquator 5 oder einem äquatornahen Breitenkreis gleich ist wie in einem polnahen Breitenkreis. Die gebildeten Strukturelemente 6 weisen eine annähernd rhombenförmige Grundfläche auf, welche durch die gekrümmten Rillen 3 begrenzt wird.

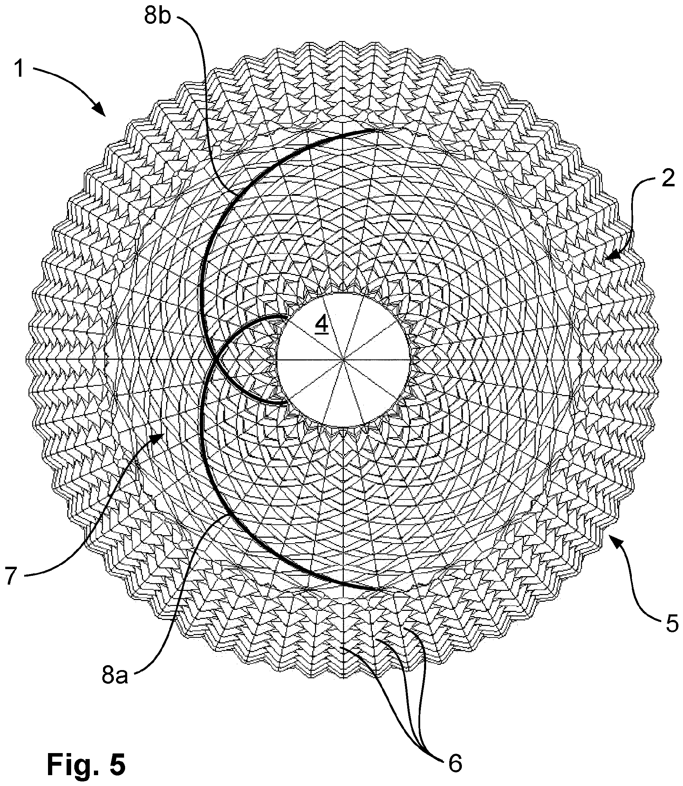

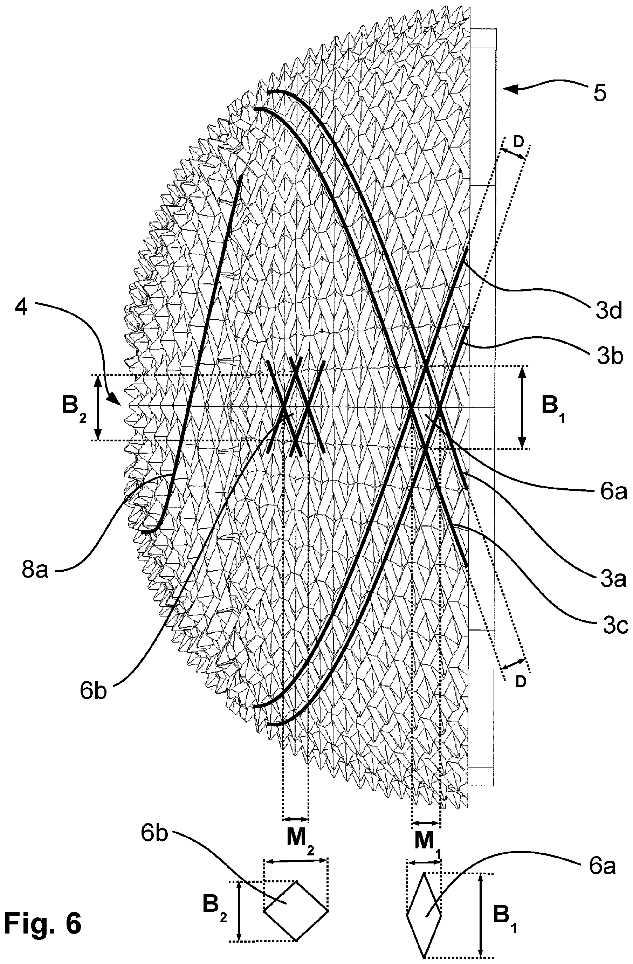

Die Figuren 5 und 6 zeigen eine Auf- und Seitenansicht einer besonders bevorzugten Ausführungsform des erfindungsgemässen Gelenkpfannenimplantats 1. Dieses Gelenkpfannenimplantat 1 umfasst zwei Bereiche mit einer Oberflächenstruktur 2, 7. Die Rillen 3a-3d der ersten Oberflächenstruktur 2 unterscheiden sich von den Rillen 8a, 8b der zweiten Oberflächenstruktur. Beispielsweise kann die Anzahl der Rillen, deren Krümmungsverlauf, Profil und/oder Tiefe unterschiedlich sein. Dadurch ergeben sich in den beiden Bereichen 2, 7 auch unterschiedliche Formen der Strukturelemente 6. Aus diesen Figuren ist der Krümmungsverlauf der Rillen 3a-3d, 8a, 8b gut ersichtlich. Ferner ist auch ersichtlich, dass der Abstand D zwischen zwei Rillen 3b und 3d mit gleicher Steigungsrichtung, d.h. der bezüglich einer Tangente einer der Rillen, z.B. Rille 3b, senkrechte Abstand zur benachbarten Rille 3d, über dies gesamte Länge der Rillen konstant ist.

Ebenso ersichtlich ist die Veränderung der Grundrisse der Strukturelemente 6 mit zunehmendem Abstand vom Äquator 5. Dabei nimmt die Länge der Diagonalen der Grundrisse parallel zu den Breitenkreisen vom Äquator in Richtung zum Pol hin ab, während die Länge der Diagonalen parallel zu den Meridianen zunimmt.

Dies ist in Fig. 6 beispielhaft an zwei Strukturelementen 6a, 6b mit annähernd rhombenförmigen Grundrissen illustriert. Da die Unterschiede der Grundrisse der beiden Strukturelement 6a, 6b in der Seitenansicht der Fig. 6 aufgrund perspektivischer Verzerrung kaum ersichtlich ist, sind zur Veranschaulichung auf der linken Seite von Fig. 6 qualitativ die beiden Strukturelemente 6a und 6b gezeigt. Die Grundrisse sind in einer senkrechten Aufsicht dargestellt. Die separate Darstellung der Grundrisse dient einzig zur Illustration einer Tendenz und ist hierzu etwas übertrieben dargestellt.

Die Grundfläche des äquatornahen Strukturelements 6a weist eine Diagonale B₁ parallel zu den Breitenkreisen auf, welche länger ist als die zu den Meridianen parallele Diagonale M₁. Mit zunehmendem Abstand zum Äquator 5 verändert sich dieses Längenverhältnis der Diagonalen. Das polnahe Strukturelement 6b weist eine zu den Breitenkreisen parallele Diagonale B₂ auf, die in der Darstellung der Fig. 6 etwa gleich lange ist, wie die zu den Meridianen parallele Diagonale M₂. Es versteht sich, dass die Diagonale B₂ auch kürzer sein kann, als Diagonale M₂.

Die Länge der zu den Breitenkreisen parallele Diagonale B₂ des polnahen Strukturelements 6b ist dabei kürzer als die entsprechende Diagonale B₁ des äquatornahen Strukturelements 6a, während die zu den Meridianen parallele Diagonale M₂ des polnahen Strukturelements 6b länger ist als die entsprechende Diagonale M₁ des äquatornahen Strukturelements 6a.

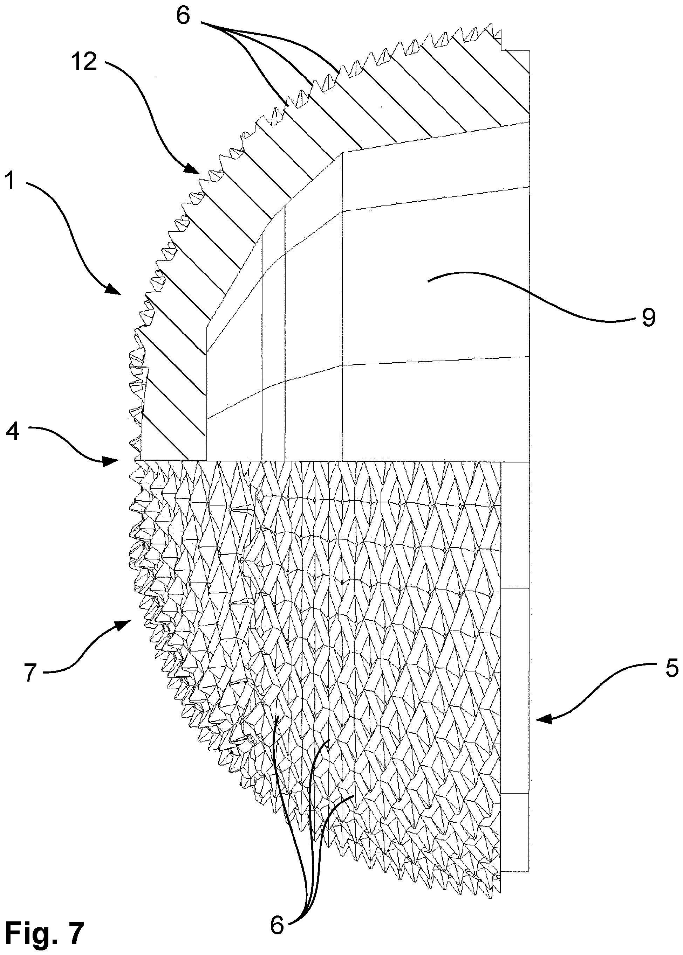

Die Figur 7 zeigt einen Teilschnitt durch ein Gelenkpfannenimplantat 1. Wie aus dieser Figur ersichtlich, weist das Gelenkpfannenimplantat 1 eine annähernd konkave Innenfläche 9 auf, welche aus mehreren, in einem Winkel zueinander stehenden Flächen besteht. Ferner weist das Gelenkpfannenimplantat 1 eine konvexe Aussenfläche 12 auf. Bei dieser Ausführungsform weist nur die konvexe Aussenfläche 12 eine Oberflächenstruktur mit Strukturelementen 6 auf.

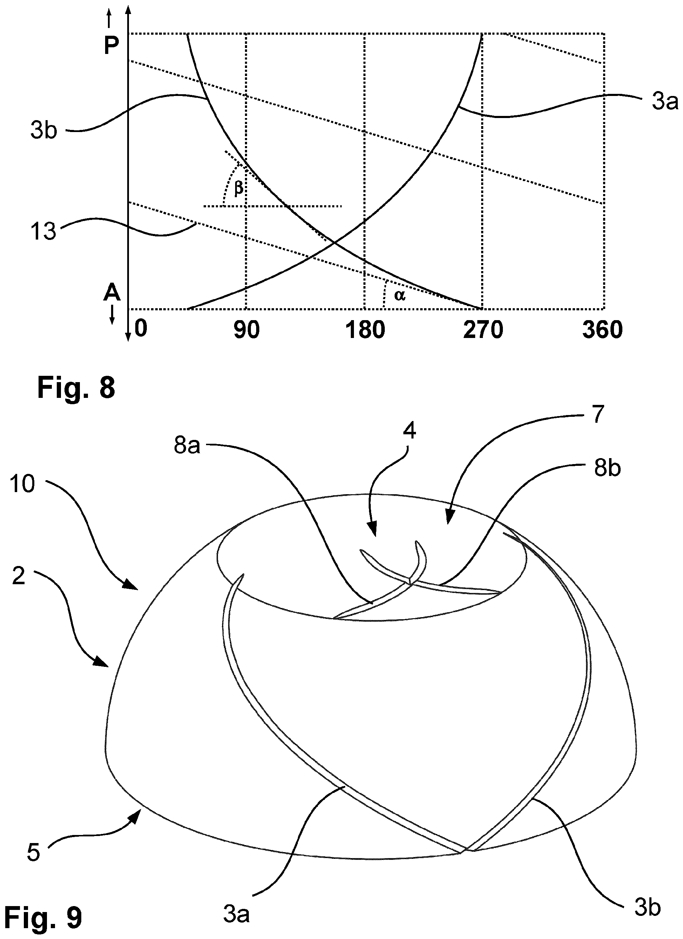

Figur 8 zeigt eine qualitative Mercator-Projektion zweier Rillen 3a, 3b auf einer Oberfläche 12 eines erfindungsgemässen Gelenkpfannenimplantats mit entgegen gesetzter Steigung. Auf der waagerechten x-Achse sind Umfangswinkel des Gelenkpfannenimplantats bezüglich einer Polachse aufgetragen. Auf der dazu senkrechten y-Achse ist ein Breitenbereich zwischen Äquator 5 und Pol 4 abgebildet. Die gestrichelten Linien parallel zur y-Achse stellen ausgewählte Längenkreise dar.

Der dargestellte Bereich ist begrenzt und erstreckt sich nicht vollständig bis zum Pol 4 bzw. bis zum Äquator 5. Kugelloxodrome, das heisst Linien auf der Kugelfläche, welche alle Breitenkreise bzw. Meridiane unter demselben Winkel schneiden, verlaufen bekanntermassen in derartigen Projektionen als Geraden. Beispielhaft ist eine Loxodrome 13 gezeigt (gestrichelte Linie), welche einen konstanten Steigungswinkel α aufweist. Im Vergleich dazu ist die zunehmende Steigung der Rillen 3 mit zunehmendem Abstand vom Äquator 5 in Richtung P zum Pol 4 hin ersichtlich.

Eine Tangente der Rille 3a schliesst in einem Bereich A zum Äquator 5 hin mit einem Breitenkreis ebenfalls den Winkel α ein. Wie sich aus dem Diagramm der Fig. 8 ergibt, nimmt die Steigung der Rille 3a zu und bricht kontinuierlich gegenüber der Loxodrome 13 in Richtung P zum Pol 4 hin weg, d.h. hat eine zunehmende Steigung. Exemplarisch ist bezüglich eines polnäheren Breitenkreises ein weiterer Winkel β gezeigt, welcher von einer Tangente im Schnittpunkt der Rille 3a mit diesem Breitenkreis eingeschlossen ist und grösser als der Winkel α ist.

Figur 9 zeigt eine Schrägansicht auf ein Zwischenerzeugnis 10 bei der Herstellung eines Gelenkpfannenimplantats 1 mit zwei. Bereichen 2, 7 für eine Oberflächenstruktur. Im den Bereichen 2, 7 sind jeweils zwei Rillen 3a und 3b (Bereich 2) und Rillen 8a und 8b (Bereich 7) ausgebildet, z.B. gefräst. Wie aus Fig. 9 ersichtlich ist, enden die sich von einem äquatornahen Bereich zum Pol 4 hin erstreckenden Rillen 3a, 3b am Übergang vom Bereich 2 zum Bereich 7 während die Rillen 8a, 8b am Übergang ansetzen und sich weiter in Richtung zum Pol 4 hin erstrecken. Ebenfalls ersichtlich ist die zunehmende Steigung der Rillen 3a, 3b bzw. 8a, 8b mit zunehmender Polnähe.

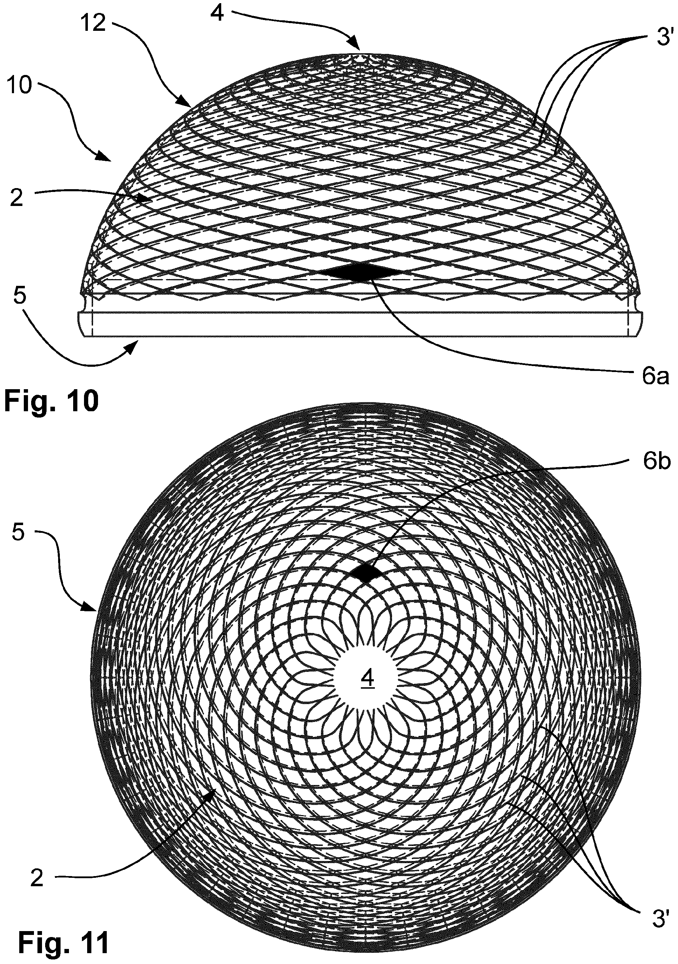

Figur 10 und 11 zeigen eine Seitenansicht bzw. eine Aufsicht eines Zwischenerzeugnisses 10 bei der Herstellung eines Gelenkpfannenimplantats 1. Auf einer äusseren Kalottenoberfläche sind Schnittlinien 3' dargestellt, längs welchen im Weiteren die Rillen 3 hergestellt werden. Besonders gut erkennbar ist in Fig. 11 ein Verlauf der Schnittlinien 3' in Polnähe, welche in den polnahen Endbereichen beinahe radial zum Pol 4 hin gerichtet sind.

Ebenso sind zur weiteren Illustration des sich mit einem Abstand vom Pol ändernden Grundrisses von später in der Herstellung durch die Rillen erzeugten Strukturelementen exemplarisch ein Grundriss eines äquatornahen Strukturelements 6a sowie eines polnahen Strukturelements 6b eingezeichnet. In der Seitenansicht der Fig. 10 ist das äquatornahe Strukturelement 6a in einer weitgehend frontalen Ansicht eingezeichnet. In der Aufsicht der Fig. 11 ist das polnahe Strukturelement 6b in weitgehend frontaler Ansicht erkennbar. Damit ergibt sich in beiden Fällen eine nur unwesentliche perspektivische Verzerrung, womit die sich mit einem Abstand vom Pol bzw. vom Äquator ändernden Grundrisse der Strukturelemente klar erkennbar sind (siehe hierzu auch Fig. 6).

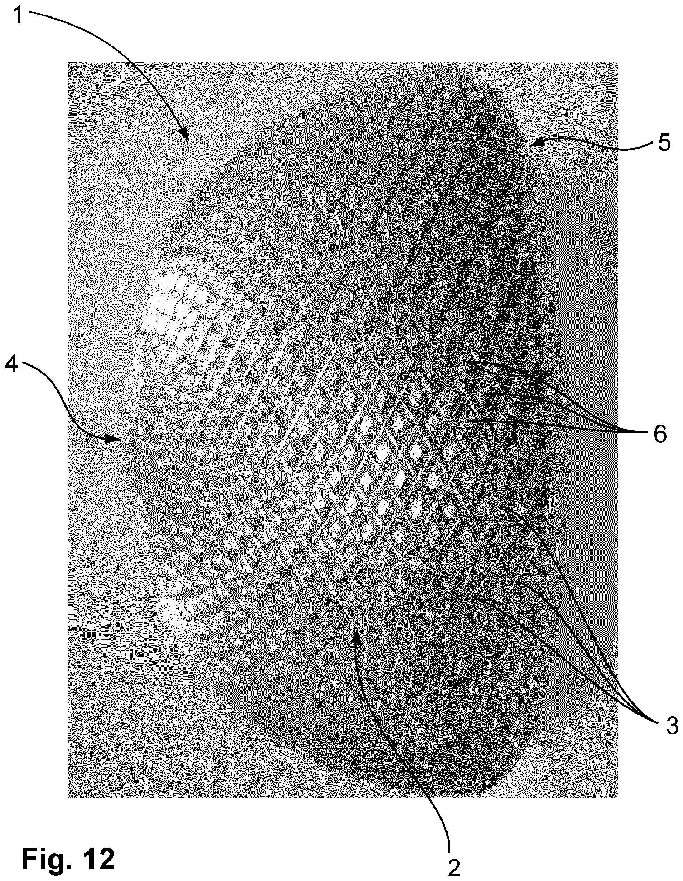

Figur 12 zeigt eine fotografische Seitenansicht eines Gelenkpfannenimplantats 1, in welcher die konstante Breite zwischen benachbarten Rillen 3 erkennbar ist. Die Strukturelemente 6 sind in dieser Ausführungsform als Pyramidenstümpfe ausgebildet.

## Patentansprüche

1. Gelenkpfannenimplantat (1), insbesondere Hüftgelenkpfannenimplantat in Form einer Kugelkalottenschale, mit mindestens einem Bereich zwischen dem Äquator und dem Pol der Kugelkalottenschale mit einer Oberflächenstruktur (2,7), welche eine Vielzahl von Strukturelementen (6) umfasst, welche durch jeweils mehrere, sich überkreuzende Rillen (3,8) mit entgegengesetzter Steigung gebildet sind, wobei die Anzahl der Strukturelemente (6) auf dem Äquator (5) oder auf einem äquatornahen Breitenkreis gleich ist wie auf einem polnahen Breitenkreis, **dadurch gekennzeichnet, dass** die Rillen (3,8) eine Krümmung derart aufweisen, dass die Steigung der Rillen gegenüber den Breitenkreisen und somit ein Winkel zwischen einer Tangente der Rillenkrümmung und einem Breitenkreis vom Äquator zum Pol hin kontinuierlich zunimmt.

2. Gelenkpfannenimplantat (1) nach Anspruch 1, **dadurch gekennzeichnet, dass** zwei benachbarte Rillen im mindestens einen Bereich mit gleicher Steigungsrichtung über ihre gesamte Länge einen konstanten Abstand zueinander aufweisen

3. Gelenkpfannenimplantat (1) nach einem der Ansprüche 1 oder 2, **dadurch gekennzeichnet, dass** die Rillen (3,8) im mindestens einen Bereich vom Äquator (5) zum Pol (4) hin eine konstante Tiefe aufweisen.

4. Gelenkpfannenimplantat nach einem der Ansprüche 1 bis 3, **dadurch gekennzeichnet, dass** der Winkel zwischen einer Tangente der Rillenkrümmung jeder Rille und einem bestimmten Breitenkreis betragsmässig gleich ist.

5. Gelenkpfannenimplantat nach einem der Ansprüche 1 bis 4, **dadurch gekennzeichnet, dass** die Strukturelemente durch benachbarte Rillen unterschiedlicher Flankenform, Rillentiefe, Rillenbreite, Rillenabstand und der Rillenzahl konfiguriert sind.

6. Gelenkpfannenimplantat (1) nach einem der Ansprüche 1 bis 5, **dadurch gekennzeichnet, dass** die Strukturelemente (6) pyramidenförmig oder pyramidenstumpfförmig ausgebildet sind.

7. Gelenkpfannenimplantat (1) nach einem der Ansprüche 1 bis 6, **dadurch gekennzeichnet, dass** die Strukturelemente (6) im meridianen Querschnitt in Richtung des Äquators (5) geneigt sind.

8. Gelenkpfannenimplantat (1) nach einem der Ansprüche 1 bis 7, **dadurch gekennzeichnet, dass** es mindestens einen zweiten Bereich mit einer Oberflächenstruktur aufweist, wobei vorzugsweise die Oberflächenstrukturen in den entsprechenden Bereichen unterschiedliche Konfigurationen aufweisen.

9. Gelenkpfannenimplantat (1) nach Anspruch 8, **dadurch gekennzeichnet, dass** die beiden Bereiche mit Oberflächenstrukturen eine unterschiedliche Anzahl von Strukturelementen aufweisen und/oder die Strukturelemente sich in ihrer Konfiguration unterscheiden.

10. Gelenkpfannenimplantat (1) nach Anspruch 7 und einem der Ansprüche 8 oder 9, **dadurch gekennzeichnet, dass** der erste Bereich in einem äquatornahen Bereich angeordnet ist und dass der zweite Bereich in einem polnahen Bereich angeordnet ist und Strukturelemente (6) in Form von Pyramiden oder Pyramidenstümpfen aufweist.

11. Gelenkpfannenimplantat (1) nach einem der Ansprüche 1 bis 10, **dadurch gekennzeichnet, dass** die Rillen (3,8) in einem äquatornahen Bereich der Oberflächenstruktur (2,7) mit einem Breitenkreis einen Winkel von kleiner als 90°, bevorzugt von 10° bis 60° bilden, vorzugsweise 15° bis 50°,wobei sich dieser Winkel zum Pol hin kontinuierlich vergrössert.

12. Gelenkpfannenimplantat (1) nach einem der Ansprüche 1 bis 11, **dadurch gekennzeichnet, dass** der mindestens eine Bereich mit einer Oberflächenstruktur (2,7) auf dem Äquator (5) oder einem äquatornahen Breitenkreis sowie einem polnahen Breitenkreis 10 bis 100 Strukturelemente (6) aufweist.

13. Gelenkpfannenimplantat (1) nach einem der Ansprüche 1 bis 12, **dadurch gekennzeichnet, dass** die Strukturelemente Grundrisse mit Seitenlängen von 0.2 - 5 mm aufweisen.

14. Gelenkpfannenimplantat (1) nach einem der Ansprüche 1 bis 13, **dadurch gekennzeichnet, dass** die Strukturelemente (6) einen annähernd rhombenförmigen Grundriss aufweisen, wobei die Länge der Diagonalen (B₁, B₂) parallel zu den Breitenkreisen vom Äquator (5) Richtung Pol (4) abnimmt, während die Länge der Diagonalen (M₁, M₂) parallel zu den Meridianen zunimmt.

15. Gelenkpfannenimplantat (1) nach einem der Ansprüche 1 bis 14, **dadurch gekennzeichnet, dass** die Rillen (3,8) einen symmetrischen oder asymmetrischen Querschnitt aufweisen.

16. Gelenkpfannenimplantat (1) nach einem der Ansprüche 1 bis 15, **dadurch gekennzeichnet, dass** sich die Rillen (3,8) nur bis zu einem polnächsten Breitenkreis erstrecken, wo der Steigungswinkel zwischen Rillen und Breitenkreis 90° erreicht.

## Claims

1. Joint socket implant (1), in particular a hip-joint socket implant in the form of a spherical cap shell, with at least one region between the equator and the pole of the spherical cap shell having a surface structure (2, 7) comprising a multiplicity of structural elements (6), which are formed by in each case a plurality of intersecting grooves (3, 8) with opposite pitch, wherein the number of the structural elements (6) on the equator (5) or on a circle of latitude near the equator is the same as on a circle of latitude near the pole, **characterized in that** the grooves (3, 8) have a curvature, in such a way that the pitch of the grooves in relation to the circles of latitude, hence an angle between a tangent of the groove curvature and a circle of latitude, increases continuously from the equator to the pole.

2. Joint socket implant (1) according to Claim 1, **characterized in that** two adjacent grooves in the at least one region with the same pitch direction are at a constant distance from each other over their entire length.

3. Joint socket implant (1) according to either of Claims 1 and 2, **characterized in that** the grooves (3, 8) in the at least one region have a constant depth from the equator (5) to the pole (4).

4. Joint socket implant according to one of Claims 1 to 3, **characterized in that** the angle between a tangent of the groove curvature of each groove and a defined circle of latitude is of identical size.

5. Joint socket implant according to one of Claims 1 to 4, **characterized in that** the structural elements are configured by adjacent grooves of different flank shape, groove depth, groove width, groove spacing and groove number.

6. Joint socket implant (1) according to one of Claims 1 to 5, **characterized in that** the structural elements (6) have the shape of a pyramid or of a truncated pyramid.

7. Joint socket implant (1) according to one of Claims 1 to 6, **characterized in that** the structural elements (6), in the meridian cross section, are inclined in the direction of the equator (5).

8. Joint socket implant (1) according to one of Claims 1 to 7, **characterized in that** it has at least one second region with a surface structure, wherein the surface structures in the corresponding regions preferably have different configurations.

9. Joint socket implant (1) according to Claim 8, **characterized in that** the two regions with surface structures have a different number of structural elements, and/or the structural elements differ in their configuration.

10. Joint socket implant (1) according to Claim 7 and either of Claims 8 and 9, **characterized in that** the first region is arranged in a region near the equator, and **in that** the second region is arranged in a region near the pole and has structural elements (6) in the form of pyramids or truncated pyramids.

11. Joint socket implant (1) according to one of Claims 1 to 10, **characterized in that** the grooves (3, 8), in a region of the surface structure (2, 7) near the equator, form an angle of less than 90°, preferably of 10° to 60°, preferably of 15° to 50°, with respect to a circle of latitude, wherein this angle increases continuously towards the pole.

12. Joint socket implant (1) according to one of Claims 1 to 11, **characterized in that** the at least one region with a surface structure (2, 7) on the equator (5) or on a circle of latitude near the equator and on a circle of latitude near the pole has 10 to 100 structural elements (6).

13. Joint socket implant (1) according to one of Claims 1 to 12, **characterized in that** the structural elements have outlines with side lengths of 0.2 - 5 mm.

14. Joint socket implant (1) according to one of Claims 1 to 13, **characterized in that** the structural elements (6) have an approximately rhomboid outline, wherein the length of the diagonals (B₁, B₂) parallel to the circles of latitude decreases from the equator (5) in the direction of the pole (4), while the length of the diagonals (M₁, M₂) parallel to the meridians increases.

15. Joint socket implant (1) according to one of Claims 1 to 14, **characterized in that** the grooves (3, 8) have a symmetrical or asymmetrical cross section.

16. Joint socket implant (1) according to one of Claims 1 to 15, **characterized in that** the grooves (3, 8) extend only as far as a circle of latitude closest to the pole, where the pitch angle between grooves and circle of latitude reaches 90°.

## Revendications

1. Implant cotyloïdien (1), en particulier implant cotyloïdien de la hanche sous la forme d'une coque à calotte sphérique, comprenant au moins une région entre l'équateur et le pôle de la coque à calotte sphérique avec une structure de surface (2, 7) qui comprend une pluralité d'éléments structurels (6) qui sont formés à chaque fois par plusieurs rainures s'intersectant (3, 8) avec des pentes opposées, le nombre des éléments structurels (6) sur l'équateur (5) ou sur un parallèle de latitude proche de l'équateur étant identique au nombre sur un parallèle de latitude proche du pôle, **caractérisé en ce que** les rainures (3, 8) présentent une courbure telle que la pente des rainures augmente par rapport aux parallèles de latitude et donc qu'un angle entre une tangente à la courbure de rainure et un parallèle de latitude augmente en continu depuis l'équateur jusqu'au pôle.

2. Implant cotyloïdien (1) selon la revendication 1, **caractérisé en ce que** deux rainures adjacentes présentent, dans au moins une région de même direction de pente, un espacement constant l'une de l'autre sur toute leur longueur.

3. Implant cotyloïdien (1) selon l'une quelconque des revendications 1 ou 2, **caractérisé en ce que** les rainures (3, 8) présentent, dans au moins une région de l'équateur (5) vers le pôle (4), une profondeur constante.

4. Implant cotyloïdien selon l'une quelconque des revendications 1 à 3, **caractérisé en ce que** l'angle entre une tangente à la courbure des rayons de chaque rainure et un parallèle de latitude déterminé est identique en valeur absolue.

5. Implant cotyloïdien selon l'une quelconque des revendications 1 à 4, **caractérisé en ce que** les éléments structurels sont configurés par des rainures adjacentes ayant des formes de flancs différentes, des profondeurs de rainure différentes, des largeurs de rainure différentes, des espacements différents entre les rainures et des nombres de rainures différents.

6. Implant cotyloïdien (1) selon l'une quelconque des revendications 1 à 5, **caractérisé en ce que** les éléments structurels (6) sont réalisés en forme de pyramide ou en forme de tronc de pyramide.

7. Implant cotyloïdien (1) selon l'une quelconque des revendications 1 à 6, **caractérisé en ce que** les éléments structurels (6) sont inclinés en section transversale méridienne dans la direction de l'équateur (5).

8. Implant cotyloïdien (1) selon l'une quelconque des revendications 1 à 7, **caractérisé en ce qu'**il présente au moins une deuxième région avec une structuration de surface, les structurations de surface présentant de préférence dans les régions correspondantes des configurations différentes.

9. Implant cotyloïdien (1) selon la revendication 8, **caractérisé en ce que** les deux régions avec des structurations de surface présentent un nombre différent d'éléments structurels et/ou les éléments structurels se distinguent de par leur configuration.

10. Implant cotyloïdien (1) selon la revendication 7 et l'une quelconque des revendications 8 ou 9, **caractérisé en ce que** la première région est disposée dans une région proche de l'équateur et **en ce que** la deuxième région est disposée dans une région proche du pôle et présente des éléments structurels (6) en forme de pyramides ou de troncs de pyramide.

11. Implant cotyloïdien (1) selon l'une quelconque des revendications 1 à 10, **caractérisé en ce que** les rainures (3, 8), dans une région proche de l'équateur de la structure de surface (2, 7), forment avec une parallèle de latitude un angle inférieur à 90°, de préférence de 10° à 60°, de préférence de 15° à 50°, cet angle augmentant en continu vers le pôle.

12. Implant cotyloïdien (1) selon l'une quelconque des revendications 1 à 11, **caractérisé en ce que** l'au moins une région avec une structuration de surface (2, 7) présente sur l'équateur (5) ou sur un parallèle de latitude proche de l'équateur ainsi que sur un parallèle de latitude proche du pôle 10 à 100 éléments structurels (6).

13. Implant cotyloïdien (1) selon l'une quelconque des revendications 1 à 12, **caractérisé en ce que** les éléments structurels présentent des projections horizontales avec des longueurs latérales de 0,2-5 mm.

14. Implant cotyloïdien (1) selon l'une quelconque des revendications 1 à 13, **caractérisé en ce que** les éléments structurels (6) présentent une projection horizontale approximativement en forme de losange, la longueur des diagonales (B₁, B₂) diminuant parallèlement au parallèle de latitude depuis l'équateur (5) dans la direction du pôle (4), tandis que la longueur des diagonales (M₁, M₂) augmente en parallèle avec les méridiens.

15. Implant cotyloïdien (1) selon l'une quelconque des revendications 1 à 14, **caractérisé en ce que** les rainures (3, 8) présentent une section transversale symétrique ou asymétrique.

16. Implant cotyloïdien (1) selon l'une quelconque des revendications 1 à 15, **caractérisé en ce que** les rainures (3, 8) s'étendent seulement jusqu'à un parallèle de latitude proche du pôle où l'angle de la pente entre les rainures et le parallèle de latitude atteint 90°.
